# EUROPEAN PATENT APPLICATION

(11) **EP 4 797 279 A1**
(43) Date of publication of application: **26.08.2026**
(21) Application number: 24879398.6
(22) Date of filing: 24.07.2024
(51) Int. Cl.: G16H 50/30

(54) **HEALTH MANAGEMENT ASSISTANCE DEVICE, HEALTH MANAGEMENT ASSISTANCE METHOD, PROGRAM, AND RECORDING MEDIUM**

(30) Priority: 20.10.2023 JP 2023180786
(71) Applicant: NEC Solution Innovators, Ltd., Koto-ku, Tokyo 136-8627 (JP)
(72) Inventor: YASUDA, Kosuke, Tokyo 136-8627 (JP); KATO, Shintaro, Tokyo 136-8627 (JP)
(74) Representative: Betten & Resch
(86) International application number: PCT/JP2024/026418
(87) International publication number: WO 2025/083968

(57) **Abstract**

An object of the present disclosure is to provide a health management assistance apparatus for supporting health management based on an individualized health index. The present disclosure provides a health management assistance apparatus, including an information acquisition part, a disease onset prediction part, a health index calculation part, and an information output part. The information acquisition part acquires disease-related information of a subject of prediction. The disease onset prediction part predicts a risk of disease onset of the subject of prediction based on the disease-related information. The health index calculation part calculates a health index individually for the subject of prediction based on the risk of disease onset. The information output part outputs disease onset prediction information based on the health index.

## Description

### INCORPORATION BY REFERENCE

This application is based upon and claims the benefit of priority from Japanese patent application No. 2023-180786, filed on October 20, 2023, the disclosure of which is incorporated herein in its entirety by reference.

### TECHNICAL FIELD

The present disclosure relates to a health management assistance apparatus, a health management assistance method, a program, and a recording medium.

### BACKGROUND ART

There is an increasing need for preventive medicine, calling for individuals to be aware of proactively managing their own health. Patent Literature 1 discloses an apparatus for predicting a prognosis of a patient with acute myocardial infarction, including an acquisition unit, a prediction unit, and an output unit, where an acquisition unit acquires at least one feature value of the patient, a prediction unit predicts the prognosis based on the at least one feature value using a trained prediction model, and an output unit outputs a prediction result of the prognosis.

### Citation List

### Patent Literature

Patent literature 1: JP 2022-184475 A

### SUMMARY

### Technical Problem

The apparatus for predicting a risk of disease onset in Patent Literature 1 predicts a risk of disease onset of an individual based on a prediction model created using sample data of a plurality of patients as training data. However, the prediction model created using sample data of a plurality of patients as training data as in Patent Literature 1, that is, an index of the risk of disease onset calculated based on statistical data, does not always apply to the individual.

An example object of the present disclosure is to provide a health management assistance apparatus, a health management assistance method, a program, and a recording medium for supporting health management based on an individualized health index.

### Solution to Problem

A health management assistance apparatus according to an example aspect of the present disclosure includes an information acquisition part, a disease onset prediction part, a health index calculation part, and an information output part. The information acquisition part acquires disease-related information of a subject of prediction. The disease onset prediction part predicts a risk of disease onset of the subject of prediction based on the disease-related information. The health index calculation part calculates a health index individually for the subject of prediction based on the risk of disease onset. The information output part outputs disease onset prediction information based on the health index.

A health management assistance method according to an example aspect of the present disclosure includes acquiring information, predicting disease onset, calculating a health index, and outputting information. The acquiring information includes acquiring disease-related information of a subject of prediction. The predicting disease onset includes predicting a risk of disease onset of the subject of prediction based on the disease-related information. The calculating a health index includes calculating a health index individually for the subject of prediction based on the risk of disease onset. The outputting information includes outputting disease onset prediction information based on the health index.

A program according to an example aspect of the present disclosure is a program for causing a computer to execute the above-described processes as procedures.

A recording medium according to an example aspect of the present disclosure is a computer-readable recording medium recorded with the program of the present disclosure. Advantageous Effects of Invention

An example advantage according to the present disclosure is that it becomes possible to support health management based on an individualized health index.

### BRIEF DESCRIPTION OF DRAWINGS

[FIG. 1] FIG. 1 is a block diagram showing an example configuration of the health management assistance apparatus of the present disclosure.
[FIG. 2] FIG. 2 is a block diagram showing an example hardware configuration of the health management assistance apparatus of the present disclosure.
[FIG. 3] FIG. 3 is a flowchart showing an example processing in the health management assistance apparatus of the present disclosure.
[FIG. 4] FIG. 4 shows an example in a case where the disease-related information includes disease-related information acquired in real time.
[FIG. 5] FIG. 5 shows an example in a case where the risk of disease onset is a risk of disease onset collected at regular intervals.
[FIG. 6] FIG. 6 shows an example of extracting data related to the disease to be predicted (heartbeat), from the disease-related information acquired in real time, and based on the extracted data, predicting the risk of disease onset of the subject of prediction by regression analysis.
[FIG. 7] FIG. 7 shows an example of the calculated health index.
[FIG. 8] FIG. 8 shows diagrams of an example of outputted statistical information.

### EXAMPLE EMBODIMENTS

The example embodiments of the present disclosure are described below with reference to the drawings. It is to be noted, however, that the present disclosure is by no means limited or restricted by the following example embodiments. In the following drawings, identical parts are indicated with identical reference signs. Regarding the descriptions of the example embodiments, reference can be made to one another unless otherwise stated. Furthermore, the configurations of the example embodiments can be combined unless otherwise stated.

### First Example Embodiment

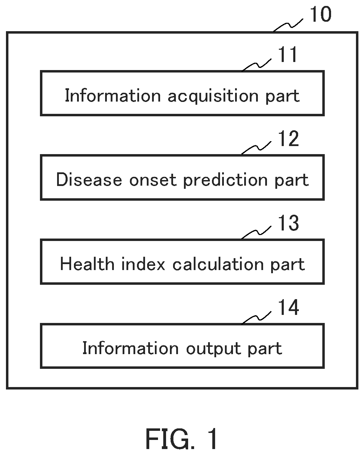

FIG. 1 is a block diagram showing an example configuration of a health management assistance apparatus 10 according to the present example embodiment (hereinafter also referred to as the "apparatus 10"). As shown in FIG. 1, the apparatus 10 includes an information acquisition part 11, a disease onset prediction part 12, a health index calculation part 13, and an information output part 14.

The apparatus 10 may be, for example, one apparatus including the aforementioned parts, or may be an apparatus to which the respective parts are connectable via a communication line network. Further, the apparatus 10 is connectable to an external device described later, via the communication line network. The communication line network is not particularly limited and may be a known network and may be, for example, wired or wireless. Examples of the communication line network include an internet line, WWW (World Wide Web), a telephone line, LAN (Local Area Network), SAN (Storage Area Network), DTN (Delay Tolerant Networking), LPWA (Low Power Wide Area), and L5G (local 5G). Examples of wireless communication include WI-FI^{®} (registered trademark), BLUETOOTH^{®} (registered trademark), local 5G, and LPWA. The wireless communication may be a form where apparatuses directly communicate with each other (Ad-Hoc communication), infrastructure communication, or may be indirect communication via an access point or the like. The apparatus 10 may be built in a server of a system, for example. The apparatus 10 may also be, for example, a personal computer (PC, e.g., a desktop PC and a laptop), a smartphone, a tablet terminal, or the like with the program of the present disclosure installed therein. The apparatus 10 may be in a form of, for example, cloud computing, edge computing, or the like such that at least one of the aforementioned parts is on a server and the rest of the parts are on a terminal.

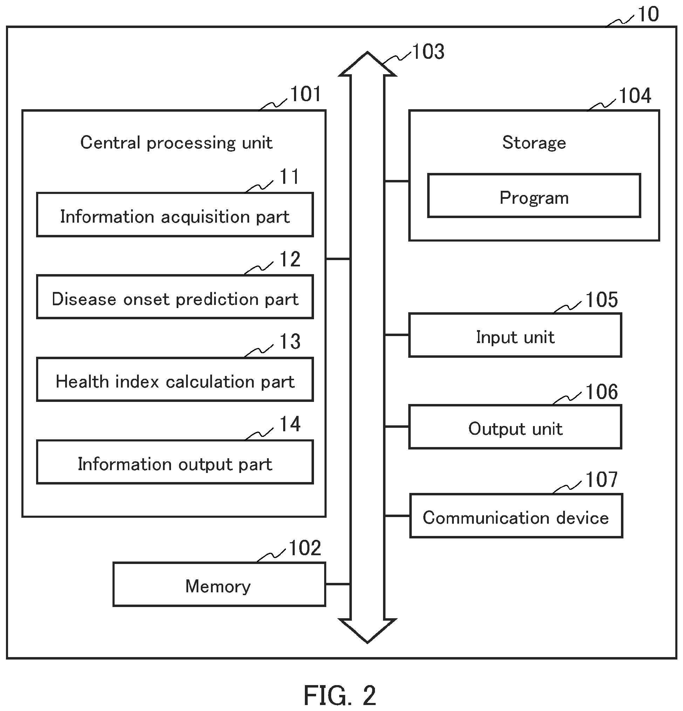

FIG. 2 is a block diagram showing an example hardware configuration of the apparatus 10. The apparatus 10 includes, for example, a central processing unit (CPU, GPU, etc.) 101, a memory 102, a bus 103, a storage 104, an input unit 105, an output unit 106, a communication device 107, and the like. The units of the apparatus 10 are connected to each other by their respective interfaces (I/F), via the bus 103.

The central processing unit 101 operates in cooperation with other configurations with a controller (a system controller, an I/O controller, or the like), and is responsible for control of the entire apparatus 10. In the apparatus 10, the central processing unit 101 executes, for example, the program of the present disclosure or other programs, and reads and writes various data. Specifically, for example, the central processing unit 101 functions as the information acquisition part 11, the disease onset prediction part 12, the health index calculation part 13, and the information output part 14. The apparatus 10 may include another arithmetic unit such as a CPU, a GPU (Graphics Processing Unit) and an APU (Accelerated Processing Unit), or a combination of these and the CPU.

The bus 103 is connectable to an external device, for example. Examples of the external device include a terminal of a user, an external storage (external data base, etc.), a printer, an external input unit, an external display, and an external imaging device. The apparatus 10 can be, for example, connected to an external network (the communication line network) through the communication device 107 connected to the bus 103, and can be connected to other devices via the external network.

The memory 102 may be, for example, a main memory (main storage). When the central processing unit 101 executes processing, for example, the memory 102 reads various operation programs such as the program of the present disclosure stored in the storage 104 to be described later, and the central processing unit 101 receives the data from the memory 102 to execute the program. The main memory is, for example, a RAM (Random-Access Memory). The memory 102 may be, for example, a ROM (Read-Only Memory).

The storage 104 is also referred to as a so-called auxiliary storage with respect to the main memory (main storage), for example. As mentioned above, the storage 104 stores operation programs including the program of the present disclosure. The storage 104 may be, for example, a combination of a recording medium and a drive for performing reading and writing on a recording medium. The recording medium is not particularly limited and may be a built-in type or an external type, and examples thereof include HD (Hard Disk), CD-ROM, CD-R, CD-RW, MO, DVD, a flash memory, and a memory card. The storage 104 may be, for example, a hard disk drive (HDD) or a solid state drive (SSD), in which a recording medium and a drive are integrated.

In the apparatus 10, the memory 102 and the storage 104 can also store various information such as log information, information acquired from an external data base (not shown) or an external device, information generated by the apparatus 10, and information used when the apparatus 10 executes processing. In this case, the memory 102 and the storage 104 may store, for example, disease-related information or the like, which will be described later. It should be noted that, at least a piece of the information may be, for example, stored in an external server other than the memory 102 and the storage 104, or may be dispersedly stored in a plurality of terminals using a blockchain technique or the like.

The apparatus 10 further includes, for example, an input unit 105 and an output unit 106. Examples of the input unit 105 include a pointing device such as a touch panel, a track pad, and a mouse; a keyboard; an imaging device such as a camera and a scanner; a card reader such as an IC card reader and a magnetic card reader; and an audio inputting device such as a microphone. Examples of the output unit 106 include a display such as an LED display and a liquid crystal display; an audio output device such as a speaker; and a printer. In the present disclosure 1, the input unit 105 and the output unit 106 are configured separately, but the input unit 105 and the output unit 106 may be configured integrally like a touch panel display.

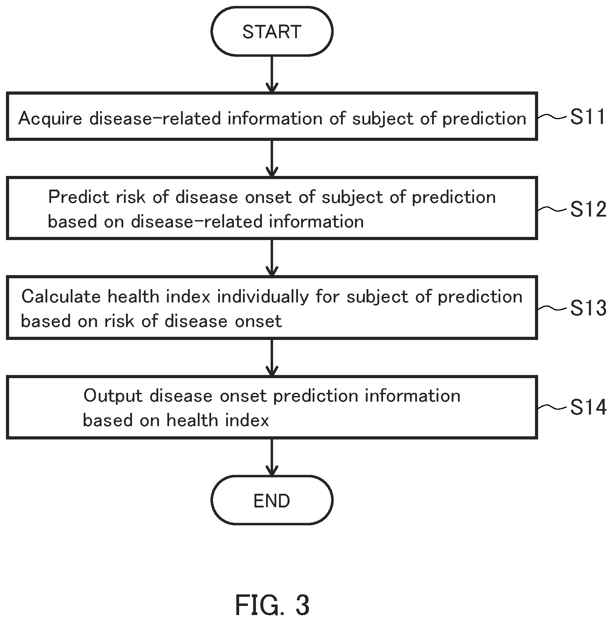

Next, an example of the health management assistance method of the present disclosure will be described with reference to the flowchart of FIG. 3. The health management assistance method of the present disclosure is performed, for example, using the apparatus 10 of FIG. 1 or FIG. 2 as described below. Note that the health management assistance method of the present disclosure is not limited to use of the apparatus 10 of FIG. 1 or FIG. 2.

First, the information acquisition part 11 acquires disease-related information of a subject of prediction (S11, acquiring information). The disease-related information includes, for example, medical record information, health checkup information, and subject attribute information (sex, age, etc.). Note that the disease-related information is not limited to the above examples, and may be in any form as long as information necessary for health management assistance can be obtained. Examples of a method for acquiring the disease-related information include a method of acquiring the information from a connected external database, a method of acquiring the information via a communication line, and a method of directly inputting required items into the apparatus 10, but are not limited thereto.

The disease-related information may include, for example, disease-related information acquired in real time. In this case, examples thereof include disease-related information collected by a wearable terminal or the like. Examples of the disease-related information collected by a wearable terminal or the like include factors for predicting the risk of disease onset described later. Examples of the wearable terminal include a terminal capable of measuring the factors, included in glasses, a hat, an earring, an earphone, a headband, a shirt, a sports bra, an armband, a bracelet, a wristwatch, a wristband, a ring, socks, or the like.

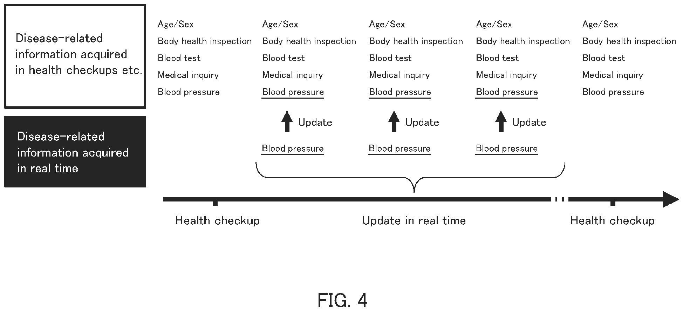

FIG. 4 shows an example in a case where the disease-related information includes disease-related information acquired in real time. First, disease-related information for predicting the risk of disease onset described later is acquired through a health checkup or the like. Usually, the acquired disease-related information is not updated until the next health checkup or the like. In contrast, if the disease-related information is acquired in real time by a wearable terminal or the like, the disease-related information can be continuously updated until the next health checkup or the like. While this example shows an example of updating blood pressure, the update of the disease-related information is not limited thereto as long as the disease-related information can be acquired in real time. As described above, by acquiring the disease-related information in real time, accuracy of the predicted risk of disease onset and a calculated health index, which will be described later, are improved.

Next, the disease onset prediction part 12 predicts a risk of disease onset of the subject of prediction based on the disease-related information (S12, predicting disease onset). The prediction of the risk of disease onset is performed, for example, using an analysis result of the risk of disease onset based on states of respective factors included in the disease-related information, based on relevancy between respective factors for predicting onset of a disease and the disease, factors having a high risk of disease onset, relevancy between the disease to be predicted and other diseases, or the like. Examples of the factors for predicting the risk of disease onset include age, sex, height, weight, high-density lipoprotein (HDL), low-density lipoprotein (LDL), triglyceride (TG), γGTP, AST (GOT), ALT (GPT), BMI, systolic blood pressure, diastolic blood pressure, blood glucose level, lifestyle habits, medical history, and medication information. Note that the factors are not limited to the above examples, and may include other additional information. Further, all of the exemplified factors are not necessarily required, and the factors may be appropriately set depending on a purpose or the like. Examples of the disease to be predicted include a brain-related disease, a heart-related disease, a liver-related disease, and a kidney-related disease, but the disease is not limited thereto and may be freely set. Examples of the brain-related disease include cerebral infarction, cerebral hemorrhage, and subarachnoid hemorrhage. Examples of the heart-related disease include myocardial infarction, angina pectoris, and heart failure. Examples of the liver-related disease to be predicted include affection of a disease such as steatotic liver disease, and hepatic cirrhosis. Examples of the kidney-related disease to be predicted include affection of a disease such as diabetic nephropathy and chronic kidney disease (CKD). The prediction of the risk of disease onset may be generated, for example, for each of a plurality of body parts. The prediction of the risk of disease onset may be, for example, a prediction of the risk of disease onset after elapse of a freely determined period.

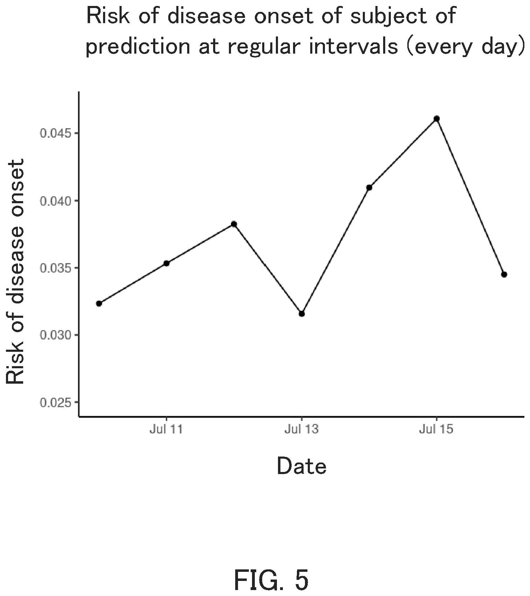

The risk of disease onset may be, for example, a risk of disease onset collected at regular intervals. Here, the term "at regular intervals" is not particularly limited, and means, for example, at regular time intervals, at regular daily intervals, or at regular weekly intervals. FIG. 5 shows an example in a case where the risk of disease onset is a risk of disease onset collected at regular intervals. In FIG. 5, a risk of disease onset of a subject of prediction is recorded every day, where the horizontal axis represents a date and the vertical axis represents a risk of onset of a disease.

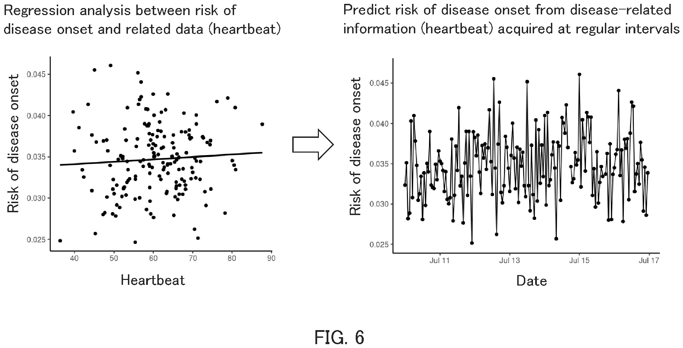

The disease onset prediction part 12 may, for example, extract data related to a disease to be predicted from the disease-related information acquired in real time, and based on the extracted data, predict the risk of disease onset of the subject of prediction. In this case, the prediction of the risk of disease onset may be, for example, prediction of a risk of disease onset by regression analysis. FIG. 6 shows an example where data related to a disease to be predicted (heartbeat) is extracted from disease-related information acquired in real time, and based on the extracted data, the risk of disease onset of the subject of prediction is predicted by regression analysis. First, the left diagram in FIG. 6 is a scatter diagram in which the horizontal axis represents data related to the disease to be predicted (heartbeat) and the vertical axis represents a risk of onset of a disease. As shown in the scatter diagram of FIG. 6, heartbeat data is extracted from the disease-related information acquired in real time, and a relationship between the data and the risk of onset of a disease is predicted by regression analysis. Based on the relationship predicted in this manner, for example, the risk of disease onset is predicted from heartbeats collected at regular intervals (at regular time intervals in the right diagram in FIG. 6).

The disease onset prediction part 12 may be, for example, a trained model (disease onset prediction model). In this case, the disease onset prediction model predicts the risk of disease onset of the subject of prediction based on the disease-related information. The disease onset prediction model is, for example, a trained model that outputs the risk of disease onset of the subject of prediction upon input of the disease-related information. The disease onset prediction model can also be regarded as, for example, a trained model for causing a computer to function so as to be trained, by machine-learning based on a plurality of pieces of disease-related information and a plurality of pieces of diagnostic information, to predict the risk of disease onset of the subject of prediction. The plurality of pieces of disease-related information may or may not necessarily include disease-related information of the subject of prediction. The plurality of pieces of diagnostic information may or may not necessarily include the disease-related information of the subject of prediction. The disease onset prediction model may be, for example, a trained model using a Cox proportional hazards model. Examples of covariates used in the Cox proportional hazards model include age, sex, height, weight, BMI, medication information (hypertension, diabetes, dyslipidemia), lifestyle habits (weight change in the past one year, weight change since age 20, smoking, exercise, daily walking and physical activity, walking speed, frequency of alcohol drinking, amount of alcohol consumption, quality of sleep, late-night meals, snacking, chewing food when eating, late dinner, skipping breakfast, and eating other than three meals of breakfast, lunch, and dinner), blood tests (HDL, LDL, TG, γGTP, AST (GOT), ALT (GPT), GLU, UA, HbA1c), and blood pressure (systolic blood pressure, diastolic blood pressure), abdominal circumference.

Next, the health index calculation part 13 calculates a health index individually for the subject of prediction based on the risk of disease onset (S13, calculating health index). The individual health index for the subject of prediction means, for example, a health index individualized for the subject of prediction. The health index may include, for example, a distribution and a threshold of the risk of disease onset. The distribution of the risk of disease onset is, for example, a normal distribution obtained based on the risk of disease onset. The threshold is, for example, a standard deviation obtained based on the risk of disease onset. The health index may be, for example, a score calculated based on the risk of disease onset. The score may be a numerical score, or may be a score ranked by symbols or the like.

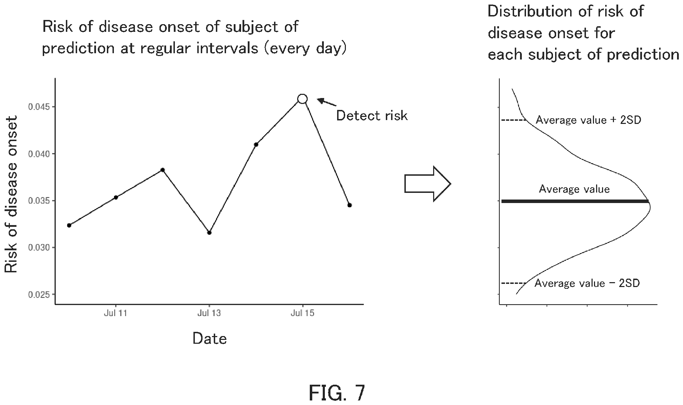

FIG. 7 shows an example of a calculated health index. As shown in the left diagram in FIG. 7, the risk of disease onset is recorded at regular intervals. As shown in the right diagram in FIG. 7, a normal distribution can be obtained based on the recorded risks of disease onset. In the case of FIG. 7, an average value ±2 SD (standard deviation) is set as a threshold. As shown in FIG. 7, when the recorded risk of disease onset deviates from the set threshold, the risk can be detected.

Thereafter, the information output part 14 outputs disease onset prediction information based on the health index (S14, outputting information), and the processing ends. The disease onset prediction information to be outputted may include, for example, the above-described distribution, threshold, score, or the like. The disease onset prediction information may be, for example, disease onset prediction information outputted based on the threshold. Examples of the disease onset prediction information outputted based on the threshold include warning information indicating deviation from the threshold, and praise information indicating non-deviation from the threshold. The warning information and the praise information may be, for example, information in a form of text, audio, or the like.

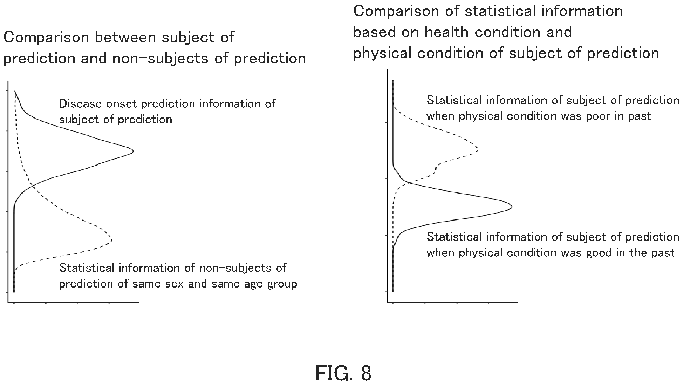

The information output part 14 may further output statistical information regarding a disease. The statistical information may be, for example, statistical information of non-subjects of prediction or statistical information of the subject of prediction. Examples of the statistical information of non-subjects of prediction include a risk of disease onset, a health index, and the like calculated based on disease-related information of non-subjects of prediction. The statistical information of non-subjects of prediction may be, for example, statistical information of an attribute same as or similar to that of the subject of prediction (e.g., age, age group, sex, etc.). The statistical information of the subject of prediction may be, for example, the health index for the subject of prediction individually calculated based on a past risk of disease onset predicted by the health management assistance apparatus of the present disclosure. The past risk of disease onset is, for example, a risk of disease onset in the past when a physical condition was good or the past when the physical condition was poor. The output may be, for example, a numerical score, a score ranked by symbols or the like, a normal distribution calculated based on the risk of disease onset calculated from the disease-related information of the non-subjects of prediction or the disease-related information of the subject of prediction, a standard deviation calculated based on the risk of disease onset of the non-subjects of prediction or based on the disease-related information of the subject of prediction, or the like. Output of the statistical information allows, for example, the subject of prediction to compare a general risk of disease onset or a general health index with the risk of disease onset or the health index of the subject of prediction. FIG. 8 shows an example of the outputted statistical information. As shown in the left diagram in FIG. 8, the disease onset prediction information of the subject of prediction and the statistical information of the non-subjects of prediction may be outputted. As shown in the right diagram in FIG. 8, as the statistical information of the subject of prediction, statistical information of the past when a physical condition was good and statistical information of the past when a physical condition was poor may be outputted. The output may be, for example, display on a dedicated monitor connected as an external apparatus, or the like. The output is not limited thereto, and may be, for example, transmission to a terminal that belongs to the subject of prediction via a communication line, output to an external database, or may be arbitrarily set depending on a purpose of use.

### Second Example Embodiment

The program according to the present disclosure is a program for causing a computer to execute the processes of the present disclosure described above. Specifically, the program according to the present disclosure is a program for causing a computer to execute, for example, an information acquisition procedure, a disease onset prediction procedure, a health index calculation procedure, and an information output procedure.

The program according to the present disclosure can also be regarded as a program that causes a computer to function as, for example, the information acquisition procedure, the disease onset prediction procedure, the health index calculation procedure, and the information output procedure.

The program according to the present disclosure can incorporate the descriptions given in the health management assistance apparatus and the health management assistance method according to the first example embodiment in the present disclosure. In each of the procedures, for example, "procedure" and "processing" can be interchangeably used. The program according to the present example embodiment may be recorded in a computer-readable recording medium, for example. The recording medium is, for example, a non-transitory computer-readable recording medium (storage medium). The recording medium is not particularly limited and examples thereof include a random access memory (RAM), a read only memory (ROM), a hard disk (HD), a flash memory (e.g., SSD (Solid State Drive), a USB flash memory, and a SD/SDHC card), an optical disc (e.g., CD-R/CD-RW, DVD-R/DVD-RW, and BD-R/BD-RE), a magnetooptical disk (MO), and a FLOPPY^{®} (registered trademark) disk (FD). The program (may be referred to as a programming product or a program product, for example) according to the present example embodiment may be in a form to be distributed from an external computer, for example. The "distribution" may be distribution via a communication line network or distribution via a wired-connected device. The program according to the present example embodiment may be executed by being installed in a device where the program is distributed, or may be executed without being installed.

While the present disclosure has been particularly shown and described with reference to example embodiments thereof, the present disclosure is not limited to these embodiments. It will be understood by those of ordinary skill in the art that various changes in form and details may be made therein without departing from the spirit and scope of the present disclosure as defined by the claims.

### SUPPLEMENTARY NOTE

The whole or part of the example embodiments disclosed above can be described as, but not limited to, the following supplementary notes.
(Supplementary Note 1)
   A health management assistance apparatus, including:
   an information acquisition part;
   a disease onset prediction part;
   a health index calculation part; and
   an information output part, wherein
   the information acquisition part acquires disease-related information of a subject of prediction,
   the disease onset prediction part predicts a risk of disease onset of the subject of prediction based on the disease-related information,
   the health index calculation part calculates a health index individually for the subject of prediction based on the risk of disease onset, and
   the information output part outputs disease onset prediction information based on the health index.
(Supplementary Note 2)
   The apparatus according to Supplementary Note 1, wherein
   the risk of disease onset is collected at regular intervals.
(Supplementary Note 3)
   The apparatus according to Supplementary Note 1 or 2, wherein
   the disease-related information includes disease-related information acquired in real time.
(Supplementary Note 4)
   The apparatus according to Supplementary Note 3, wherein
   the disease onset prediction part extracts data related to a disease to be predicted, from the disease-related information acquired in real time, and based on the extracted data, predicts the risk of disease onset of the subject of prediction.
(Supplementary Note 5)
   The apparatus according to any one of Supplementary Notes 1 to 4, wherein the health index includes a distribution and a threshold of the risk of disease onset.
(Supplementary Note 6)
   The apparatus according to Supplementary Note 5, wherein
   the disease onset prediction information is outputted based on the threshold.
(Supplementary Note 7)
   The apparatus according to any one of Supplementary Notes 1 to 6, wherein the information output part further outputs statistical information of a disease.
(Supplementary Note 8)
   The apparatus according to Supplementary Note 7, wherein
   the statistical information is statistical information of non-subjects of prediction, or statistical information of the subject of prediction.
(Supplementary Note 9)
   A health management assistance method, including:
   acquiring information;
   predicting disease onset;
   calculating a health index; and
   outputting information, wherein
   the acquiring information includes acquiring disease-related information of a subject of prediction,
   the predicting disease onset includes predicting a risk of disease onset of the subject of prediction based on the disease-related information,
   the calculating a health index includes calculating a health index individually for the subject of prediction based on the risk of disease onset, and
   the outputting information includes outputting disease onset prediction information based on the health index.
(Supplementary Note 10)
   The method according to Supplementary Note 9, wherein
   the risk of disease onset is collected at regular intervals.
(Supplementary Note 11)
   The method according to Supplementary Note 9 or 10, wherein
   the disease-related information includes disease-related information acquired in real time.
(Supplementary Note 12)
   The method according to Supplementary Note 11, wherein
   the predicting disease onset includes extracting data related to a disease to be predicted, from the disease-related information acquired in real time, and based on the extracted data, predicting the risk of disease onset of the subject of prediction.
(Supplementary Note 13)
   The method according to any one of Supplementary Notes 9 to 12, wherein
   the health index includes a distribution and a threshold of the risk of disease onset.
(Supplementary Note 14)
   The method according to Supplementary Note 13, wherein
   the disease onset prediction information is outputted based on the threshold.
(Supplementary Note 15)
   The method according to any one of Supplementary Notes 9 to 14, wherein the outputting information further includes outputting statistical information of a disease.
(Supplementary Note 16)
   The method according to Supplementary Note 15, wherein
   the statistical information is statistical information of non-subjects of prediction, or statistical information of the subject of prediction.
(Supplementary Note 17)
   A program for causing a computer to execute the following procedures, including:
   an information acquisition procedure;
   a disease onset prediction procedure;
   a health index calculation procedure; and
   an information output procedure, wherein
   the information acquisition procedure includes acquisition of disease-related information of a subject of prediction,
   the disease onset prediction procedure includes prediction of a risk of disease onset of the subject of prediction based on the disease-related information,
   the health index calculation procedure includes calculation of a health index individual for the subject of prediction based on the risk of disease onset, and
   the information output procedure includes output of disease onset prediction information based on the health index.
(Supplementary Note 18)
   The program according to Supplementary Note 17, wherein
   the risk of disease onset is collected at regular intervals.
(Supplementary Note 19)
   The program according to Supplementary Note 17 or 18, wherein
   the disease-related information includes disease-related information acquired in real time.
(Supplementary Note 20)
   The program according to Supplementary Note 19, wherein
   the disease onset prediction procedure includes extracting data related to a disease to be predicted, from the disease-related information acquired in real time, and based on the extracted data, predicting the risk of disease onset of the subject of prediction.
(Supplementary Note 21)
   The program according to any one of Supplementary Notes 17 to 20, wherein the health index includes a distribution and a threshold of the risk of disease onset.
(Supplementary Note 22)
   The program according to Supplementary Note 21, wherein
   the disease onset prediction information is outputted based on the threshold.
(Supplementary Note 23)
   The program according to any one of Supplementary Notes 17 to 22, wherein the information output procedure further includes output of statistical information of a disease.
(Supplementary Note 24)
   The program according to Supplementary Note 23, wherein
   the statistical information is statistical information of non-subjects of prediction, or statistical information of the subject of prediction.
(Supplementary Note 25)
   A computer-readable recording medium recorded with a program for causing a computer to execute the following procedures, including:
   an information acquisition procedure;
   a disease onset prediction procedure;
   a health index calculation procedure; and
   an information output procedure, wherein
   the information acquisition procedure includes acquisition of disease-related information of a subject of prediction,
   the disease onset prediction procedure includes prediction of a risk of disease onset of the subject of prediction based on the disease-related information,
   the health index calculation procedure includes calculation of a health index individual for the subject of prediction based on the risk of disease onset, and
   the information output procedure includes output of disease onset prediction information based on the health index.
(Supplementary Note 26)
   The recording medium according to Supplementary Note 25, wherein
   the risk of disease onset is collected at regular intervals.
(Supplementary Note 27)
   The recording medium according to Supplementary Note 25 or 26, wherein
   the disease-related information includes disease-related information acquired in real time.
(Supplementary Note 28)
   The recording medium according to Supplementary Note 27, wherein
   the disease onset prediction procedure includes extracting data related to a disease to be predicted, from the disease-related information acquired in real time, and based on the extracted data, predicting the risk of disease onset of the subject of prediction.
(Supplementary Note 29)
   The recording medium according to any one of Supplementary Notes 25 to 28, wherein
   the health index includes a distribution and a threshold of the risk of disease onset.
(Supplementary Note 30)
   The recording medium according to Supplementary Note 29, wherein
   the disease onset prediction information is outputted based on the threshold.
(Supplementary Note 31)
   The recording medium according to any one of Supplementary Notes 25 to 30, wherein
   the information output procedure further includes output of statistical information of a disease.
(Supplementary Note 32)
   The recording medium according to Supplementary Note 31, wherein
   the statistical information is statistical information of non-subjects of prediction, or statistical information of the subject of prediction.

### Industrial Applicability

An example advantage according to the present disclosure is that it becomes possible to support health management based on an individualized health index. The field to which the present disclosure can be applied is not limited, and the present disclosure can be applied to a wide range of fields using a health management assistance apparatus.

### Reference Signs List

- 10:: health management assistance apparatus
- 11:: information acquisition part
- 12:: disease onset prediction part
- 13:: health index calculation part
- 14:: information output part
- 101:: central processing unit
- 102:: memory
- 103:: bus
- 104:: storage
- 105:: input unit
- 106:: output unit
- 107:: communication device

## Claims

1. A health management assistance apparatus, comprising:
an information acquisition part;
a disease onset prediction part;
a health index calculation part, and
an information output part, wherein
the information acquisition part acquires disease-related information of a subject of prediction,
the disease onset prediction part predicts a risk of disease onset of the subject of prediction based on the disease-related information,
the health index calculation part calculates a health index individually for the subject of prediction based on the risk of disease onset, and
the information output part outputs disease onset prediction information based on the health index.

2. The apparatus according to claim 1, wherein
the risk of disease onset is collected at regular intervals.

3. The apparatus according to claim 1 or 2, wherein
the disease-related information includes disease-related information acquired in real time.

4. The apparatus according to claim 3, wherein
the disease onset prediction part extracts data related to a disease to be predicted, from the disease-related information acquired in real time, and based on the extracted data, predicts the risk of disease onset of the subject of prediction.

5. The apparatus according to claim 1 or 2, wherein
the health index includes a distribution and a threshold of the risk of disease onset.

6. The apparatus according to claim 5, wherein
the disease onset prediction information is outputted based on the threshold.

7. The apparatus according to claim 1 or 2, wherein
the information output part further outputs statistical information of a disease.

8. The apparatus according to claim 7, wherein
the statistical information is statistical information of non-subjects of prediction, or statistical information of the subject of prediction.

9. A health management assistance method, comprising:
acquiring information;
predicting disease onset;
calculating a health index, and
outputting information, wherein
the acquiring information includes acquiring disease-related information of a subject of prediction,
the predicting disease onset includes predicting a risk of disease onset of the subject of prediction based on the disease-related information,
the calculating a health index includes calculating a health index individually for the subject of prediction based on the risk of disease onset, and
the outputting information includes outputting disease onset prediction information based on the health index.

10. The method according to claim 9, wherein
the risk of disease onset is collected at regular intervals.

11. The method according to claim 9 or 10, wherein
the disease-related information includes disease-related information acquired in real time.

12. The method according to claim 11, wherein
the predicting disease onset includes extracting data related to a disease to be predicted, from the disease-related information acquired in real time, and based on the extracted data, predicting the risk of disease onset of the subject of prediction.

13. The method according to claim 9 or 10, wherein
the health index includes a distribution and a threshold of the risk of disease onset.

14. The method according to claim 13, wherein
the disease onset prediction information is outputted based on the threshold.

15. The method according to claim 9 or 10, wherein
the outputting information further includes outputting statistical information of a disease.

16. The method according to claim 15, wherein
the statistical information is statistical information of non-subjects of prediction, or statistical information of the subject of prediction.

17. A program for causing a computer to execute the following procedures,
comprising:
an information acquisition procedure;
a disease onset prediction procedure;
a health index calculation procedure, and
an information output procedure, wherein
the information acquisition procedure includes acquisition of disease-related information of a subject of prediction,
the disease onset prediction procedure includes prediction of a risk of disease onset of the subject of prediction based on the disease-related information,
the health index calculation procedure includes calculation of a health index individual for the subject of prediction based on the risk of disease onset, and
the information output procedure includes output of disease onset prediction information based on the health index.

18. The program according to claim 17, wherein
the risk of disease onset is collected at regular intervals.

19. The program according to claim 17 or 18, wherein
the disease-related information includes disease-related information acquired in real time.

20. A computer-readable recording medium recorded with a program for causing a computer to execute the following procedures, comprising:
an information acquisition procedure;
a disease onset prediction procedure;
a health index calculation procedure, and
an information output procedure, wherein
the information acquisition procedure includes acquisition of disease-related information of a subject of prediction,
the disease onset prediction procedure includes prediction of a risk of disease onset of the subject of prediction based on the disease-related information,
the health index calculation procedure includes calculation of a health index individual for the subject of prediction based on the risk of disease onset, and
the information output procedure includes output of disease onset prediction information based on the health index.
